# EUROPÄISCHE PATENTSCHRIFT

(11) **EP 4 031 894 B1**
(45) Veröffentlichungstag und Bekanntmachung des Hinweises auf die Patenterteilung: **07.06.2023**
(21) Anmeldenummer: 20772042.6
(22) Anmeldetag: 14.09.2020
(51) Int. Cl.: G01R 33/56, A61B 5/055, G06T 5/00, G06T 7/00, A61B 5/00, G06N 3/04, G06N 3/08, A61K 49/10

(54) **VORHERSAGE VON MRT-AUFNAHMEN DURCH EIN MITTELS ÜBERWACHTEN LERNENS TRAINIERTES VORHERSAGEMODELL**
PREDICTING MRI IMAGES BY PREDICTION MODEL TRAINED BY SUPERVISED LEARNING
PRÉDICTION DES IMAGES IRM PAR MODÈLE DE PRÉDICTION FORMÉ PAR APPRENTISSAGE SUPERVISÉ

(30) Priorität: 18.09.2019 EP 19197989
(43) Veröffentlichungstag der Anmeldung: 27.07.2022
(60) Teilanmeldung: 23163651.5
(73) Patentinhaber: Bayer Aktiengesellschaft, 51373 Leverkusen (DE)
(72) Erfinder: ROHRER, Martin, 12203 Berlin (DE); UBER III, Arthur, Pittsburgh, PA 15208 (US)
(74) Vertreter: BIP Patents
(86) Internationale Anmeldenummer: PCT/EP2020/075593
(87) Internationale Veröffentlichungsnummer: WO 2021/052896

(56) Entgegenhaltungen:
- WEIZMAN LIOR ET AL: "Prediction of Brain MR Scans in Longitudinal Tumor Follow-Up Studies", 1. Oktober 2012 (2012-10-01), BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, XP047463777, ISBN: 978-3-642-17318-9 Abstract, drittletzter Absatz des Kapitels 1, Kapitel 2, die ersten zwei Absätze des Kapitels 3, vorletzter Absatz des Kapitels 4; Abbildungen 1-3
- Alon Shtern: "Shape Correspondence using Spectral Methods and Deep Learning", , 30. August 2017 (2017-08-30), XP055754117, http://www.cs.technion.ac.il/users/wwwb/cg i-bin/tr-info.cgi/2017/PHD/PHD-2017-13 Gefunden im Internet: URL:http://www.cs.technion.ac.il/users/www b/cgi-bin/tr-get.cgi/2017/PHD/PHD-2017-13. pdf [gefunden am 2020-11-25]
- KWON HEON-JU ET AL: "Differentiation of small (<=2 cm) hepatocellular carcinomas from small benign nodules in cirrhotic liver on gadoxetic acid-enhanced and diffusion-weighted magnetic resonance ima", ABDOMINAL IMAGING, NEW YORK, US, Bd. 40, Nr. 1, 6. Juli 2014 (2014-07-06), Seiten 64-75, XP035417363, ISSN: 0942-8925, DOI: 10.1007/S00261-014-0188-8 [gefunden am 2014-07-06]
- ANGSHUL MAJUMDAR: "Motion predicted online dynamic MRI reconstruction from partially sampled k-space data", MAGNETIC RESONANCE IMAGING, Bd. 31, Nr. 9, 25. Juli 2013 (2013-07-25), Seiten 1578-1586, XP055753692, TARRYTOWN, NY, US ISSN: 0730-725X, DOI: 10.1016/j.mri.2013.06.003
- TAKESHIMA, HIDENORI: "Integrating Spatial and Temporal Correlations into a Deep Neural Network for Low-delay Reconstruction of Highly Undersampled Radial Dynamic Images", INTERNATIONAL SOCIETY FOR MAGNETIC RESONANCE IN MEDICINE, ISMRM, 2030 ADDISON STREET, 7TH FLOOR, BERKELEY, CA 94704 USA, Nr. 2796, 1. Juni 2018 (2018-06-01), XP040702004,
- TAKESHIMA, HIDENORI: "Integrating Spatial and Temporal Correlations into a Deep Neural Network for Low-delay Reconstruction of Highly Undersampled Radial Dynamic Images", INTERNATIONAL SOCIETY FOR MAGNETIC RESONANCE IN MEDICINE, ISMRM, 2030 ADDISON STREET, 7TH FLOOR, BERKELEY, CA 94704 USA, no. 2796, 1 June 2018 (2018-06-01), XP040702004,

## Beschreibung

Die vorliegende Erfindung befasst sich mit der Beschleunigung von MRT-Untersuchungen, insbesondere bei der Detektion und Differentialdiagnose fokaler Leberläsionen mittels dynamischer kontrastverstärkender Magnetresonanztomographie (MRT). Gegenstände der vorliegenden Erfindung sind ein Verfahren, ein System und ein Computerprogrammprodukt zur Vorhersage von MRT-Aufnahmen, insbesondere der Leber während der hepatobiliären Phase.

Die Magnetresonanztomographie, abgekürzt MRT oder MR (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen. Eine Verkürzung der T1-Zeit führt zu einer Zunahme der Signalintensität in T1-gewichteten MRT-Aufnahmen, eine Verkürzung der T2-Zeit führt zu einer Abnahme der Signalintensität in T2-gewichteten MRT-Aufnahmen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

Ein Beispiel für ein superparamagnetisches Kontrastmittel sind Eisenoxidnanopartikel (SPIO, engl.: *superparamagnetic iron oxide*)*.*

Beispiele für paramagnetische Kontrastmittel sind Gadolinium-Chelate wie Gadopentetat-Dimeglumin (Handelsname: Magnevist^{®} u.a.), Gadobenat-Dimeglumin (Handelsname: Multihance^{®}), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

Primovist^{®} kann zur Detektion von Tumoren in der Leber eingesetzt werden. Die Blutversorgung des gesunden Lebergewebes erfolgt in erster Linie über die Pfortader (*Vena portae*)*,* während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist^{®} kann zur Erkennung gutartiger und bösartiger fokaler Leberläsionen eingesetzt werden. Es liefert mittels T 1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Die mittels Primovist^{®} erzielte Kontrastverstärkung kann man in mindestens zwei Phasen unterteilen: in eine dynamische Phase (umfassend die so genannte arterielle Phase, portalvenöse Phase und Spätphase) und die hepatobiliäre Phase, in der bereits eine signifikante Aufnahme von Primovist^{®} in die Hepatozyten stattgefunden hat.

Bei der durch Primovist^{®} erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist^{®} 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Die zeitliche Verfolgung der Verteilung des Kontrastmittels über die dynamische Phase und die hepatobiliäre Phase bietet auf der einen Seite eine gute Möglichkeit der Detektion und Differentialdiagnose fokaler Leberläsionen; auf der anderen Seite zieht sich die Untersuchung jedoch über eine vergleichsweise lange Zeitspanne hin. Über diese Zeitspanne sollten Bewegungen des Patienten vermieden werden, um Bewegungsartefakte in den MRT-Aufnahme zu minimieren. Die lang andauernde Bewegungseinschränkung kann für einen Patienten unangenehm sein.

Ausgehend vom beschriebenen Stand der Technik bestand die technische Aufgabe darin, die Untersuchung für den Patienten weniger unangenehm zu gestalten.

Diese Aufgabe wird durch die Gegenstände der unabhängigen Patentansprüche gelöst. Bevorzugte Ausführungsformen der vorliegenden Erfindung finden sich in den abhängigen Patentansprüchen, in dieser Beschreibung und in den Zeichnungen. Mittels der vorliegenden Erfindung wird die Zeitspanne der MRT-Untersuchung erheblich reduziert, was für einen Patienten eine Erleichterung darstellt.

Ein erster Gegenstand der vorliegenden Erfindung ist ein computer-implementiertes Verfahren gemäß Anspruch 1 umfassend die Schritte
- Empfangen einer Mehrzahl von ersten MRT-Aufnahmen, wobei zumindest ein Teil der ersten MRT-Aufnahmen einen Untersuchungsbereich während einer ersten Zeitspanne nach einer Applikation eines Kontrastmittels zeigt,
- Zuführen der Mehrzahl von ersten MRT-Aufnahmen einem Vorhersagemodell, wobei das Vorhersagemodell anhand von Referenz-MRT-Aufnahmen mittels überwachten Lernens trainiert worden ist, aus ersten Referenz-MRT-Aufnahmen, von denen zumindest ein Teil einen Untersuchungsbereich während einer ersten Zeitspanne nach einer Applikation eines Kontrastmittels zeigt, eine oder mehrere zweite Referenz-MRT-Aufnahmen zu erzeugen, die den Untersuchungsbereich während einer zweiten Zeitspanne zeigen, wobei die zweite Zeitspanne zeitlich auf die erste Zeitspanne folgt,
- Erzeugen einer oder mehrerer vorhergesagter MRT-Aufnahmen, die den Untersuchungsbereich während einer zweiten Zeitspanne zeigen, durch das Vorhersagemodell, wobei die zweite Zeitspanne zeitlich auf die erste Zeitspanne folgt,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher, wobei der Untersuchungsbereich eine Leber oder ein Teil einer Leber eines Menschen ist, wobei das Kontrastmittel ein hepatobiliäres Kontrastmittel ist, wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert, und wobei die zweite Zeitspanne mindestens 10 Minuten nach der Applikation des Kontrastmittels beginnt.

Ein weiterer Gegenstand der vorliegenden Erfindung ist ein System gemäß Anspruch 10 umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, eine Mehrzahl von ersten MRT-Aufnahmen zu empfangen, wobei zumindest ein Teil der ersten MRT-Aufnahmen einen Untersuchungsbereich während einer ersten Zeitspanne nach einer Applikation eines Kontrastmittels zeigt,
   - wobei die Steuer- und Recheneinheit konfiguriert ist, anhand der empfangenen ersten MRT-Aufnahmen eine oder mehrere zweite MRT-Aufnahmen mittels eines Vorhersagemodells vorherzusagen, wobei die eine oder die mehreren zweiten MRT-Aufnahmen den Untersuchungsbereich während einer zweiten Zeitspanne zeigen, wobei die zweite Zeitspanne zeitlich auf die erste Zeitspanne folgt,
   - wobei die Steuer- und Recheneinheit konfiguriert ist, die Ausgabeeinheit zu veranlassen, die eine oder die mehreren zweiten MRT-Aufnahmen anzuzeigen, auszugeben oder in einem Datenspeicher zu speichern,
wobei das Vorhersagemodell mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt, wobei der Untersuchungsbereich eine Leber oder ein Teil einer Leber eines Menschen ist, wobei das Kontrastmittel ein hepatobiliäres Kontrastmittel ist, wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert, und wobei die zweite Zeitspanne mindestens 10 Minuten nach der Applikation des Kontrastmittels beginnt.

Ein weiterer Gegenstand der vorliegenden Erfindung ist ein Computerprogrammprodukt gemäß Anspruch 11 umfassend ein

Computerprogramm, das in einen Arbeitsspeicher eines Computersystems geladen werden kann und dort das Computersystem dazu veranlasst, folgende Schritte ausführen:
- Empfangen einer Mehrzahl von ersten MRT-Aufnahmen, wobei zumindest ein Teil der ersten MRT-Aufnahmen einen Untersuchungsbereich während einer ersten Zeitspanne nach einer Applikation eines Kontrastmittels zeigt,
- Zuführen der Mehrzahl von ersten MRT-Aufnahmen einem Vorhersagemodell, wobei das Vorhersagemodell anhand von Referenz-MRT-Aufnahmen mittels überwachten Lernens trainiert worden ist, aus ersten Referenz-MRT-Aufnahmen, von denen zumindest ein Teil einen Untersuchungsbereich während einer ersten Zeitspanne nach einer Applikation eines Kontrastmittels zeigt, eine oder mehrere zweite Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
- Erzeugen einer oder mehrerer vorhergesagter MRT-Aufnahmen, die den Untersuchungsbereich während einer zweiten Zeitspanne zeigen, durch das Vorhersagemodell, wobei die zweite Zeitspanne zeitlich auf die erste Zeitspanne folgt,
- Anzeigen und/oder Ausgeben der einen oder der mehreren vorhergesagten MRT-Aufnahmen und/oder Speichern der einen oder der mehreren vorhergesagten MRT-Aufhahmen in einem Datenspeicher,
wobei das Vorhersagemodell mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt, wobei der Untersuchungsbereich eine Leber oder ein Teil einer Leber eines Menschen ist, wobei das Kontrastmittel ein hepatobiliäres Kontrastmittel ist, wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert, und wobei die zweite Zeitspanne mindestens 10 Minuten nach der Applikation des Kontrastmittels beginnt.

Ein weiterer Gegenstand der vorliegenden Erfindung ist die Verwendung eines Kontrastmittels in einem MRT-Verfahren nachdem ein Kontrastmittel appliziert wurde und sich in einem Untersuchungsbereich verteilt hat, gemäß Anspruch 12, wobei das MRT-Verfahren die folgenden Schritte umfasst:
- Erzeugen einer Mehrzahl von ersten MRT-Aufnahmen von dem Untersuchungsbereich während einer ersten Zeitspanne
- Zuführen der erzeugten ersten MRT-Aufnahmen einem Vorhersagemodell, wobei das Vorhersagemodell anhand von Referenz-MRT-Aufnahmen mittels überwachten Lernens trainiert worden ist, aus ersten Referenz-MRT-Aufnahmen, die einen Untersuchungsbereich während einer ersten Zeitspanne zeigen, eine oder mehrere zweite Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
- Empfangen einer oder mehrerer vorhergesagter MRT-Aufnahmen, die den Untersuchungsbereich während einer zweiten Zeitspanne zeigen, als Ausgabe von dem Vorhersagemodel, wobei die zweite Zeitspanne zeitlich auf die erste Zeitspanne folgt,
- Anzeigen und/oder Ausgeben der einen oder der mehreren vorhergesagten MRT-Aufnahmen und/oder Speichern der einen oder der mehreren vorhergesagten MRT-Aufhahmen in einem Datenspeicher,
wobei der Untersuchungsbereich eine Leber oder ein Teil einer Leber eines Menschen ist, wobei das Kontrastmittel ein hepatobiliäres Kontrastmittel ist, wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert, und wobei die zweite Zeitspanne mindestens 10 Minuten nach der Applikation des Kontrastmittels beginnt.

Ein weiterer Gegenstand ist ein Kit gemäß Anspruch 13 umfassend ein Kontrastmittel und das erfindungsgemäße Computerprogrammprodukt, wobei das Kontrastmittel ein hepatobiliäres MRT-Kontrastmittel ist.

Die Erfindung wird nachstehend näher erläutert, ohne zwischen den Erfindungsgegenständen (Verfahren, System, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kit) sie erfolgen Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung verkürzt die Zeitspanne der Untersuchung eines Untersuchungsobjekts bei der Erzeugung von MRT-Aufnahmen. Dies wird dadurch erreicht, dass MRT-Aufnahmen von einem Untersuchungsbereich des Untersuchungsobjekts in einer ersten Zeitspanne gemessen werden (Magnetresonanzmessung), und die gemessenen MRT-Aufnahmen dann verwendet werden, um eine oder mehrere MRT-Aufnahmen, die den Untersuchungsbereich in einer zweiten Zeitspanne zeigen, mit Hilfe eines selbstlernenden Algorithmus vorherzusagen. Die eigentliche Magnetresonanzmessung am Untersuchungsobjekt beschränkt sich somit auf die erste Zeitspanne und umfasst nicht die zweite Zeitspanne. Die MRT-Aufnahmen, die den Untersuchungsbereich während der ersten Zeitspanne zeigen, beinhalten Informationen, die eine Vorhersage für die zweite Zeitspanne erlauben.

Das Untersuchungsobjekt ist üblicherweise ein Lebewesen, vorzugsweise ein Säugetier, erfindungsgemäß ist das Untersuchungsobjekt ein Mensch. Der Untersuchungsbereich ist ein Teil des Untersuchungsobjekts, zum Beispiel ein Organ oder ein Teil eines Organs. Erfindungsgemäß handelt es sich bei dem Untersuchungsbereich um die Leber oder um einen Teil der Leber eines Menschen.

Der Untersuchungsbereich, auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird in ein Grundmagnetfeld eingebracht. Der Untersuchungsbereich wird einem MRT-Verfahren unterzogen und dabei wird eine Mehrzahl von MRT-Aufnahmen erzeugt, die den Untersuchungsbereich während einer ersten Zeitspanne zeigen. Diese während der ersten Zeitspanne messtechnisch erzeugten MRT-Aufnahmen werden in dieser Beschreibung auch als erste MRT-Aufnahmen bezeichnet.

Der Begriff Mehrzahl bedeutet, dass mindestens zwei (erste) MRT-Aufnahmen, vorzugsweise mindestens drei (erste), ganz besonders bevorzugt mindestens vier (erste) MRT-Aufnahmen erzeugt werden.

Dem Untersuchungsobjekt wird ein Kontrastmittel verabreicht, das sich in dem Untersuchungsbereich verteilt. Das Kontrastmittel wird vorzugsweise intravenös als Bolus gewichtsadaptiert verabreicht (zum Beispiel in eine Armvene).

Unter einem Kontrastmittel wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Relaxationszeit und/oder der T2-Relaxationszeit.

Erfindungsgemäß ist das Kontrastmittel ein hepatobiliäres Kontrastmittel wie beispielsweise Gd-EOB-DTPA oder Gd-BOPTA.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Erfindungsgemäß beginnt die erste Zeitspanne vor der Applikation des Kontrastmittels oder mit der Applikation des Kontrastmittels. Vorteilhaft ist es, wenn eine oder mehrere MRT-Aufnahmen erzeugt werden, die den Untersuchungsbereich ohne Kontrastmittel zeigen (native Aufnahmen), da ein Radiologe in solchen Aufnahmen bereits wichtige Informationen zum Gesundheitszustand des Untersuchungsobjekts gewinnen kann. Beispielsweise kann ein Radiologe in solchen nativen MRT-Aufnahmen Blutungen erkennen.

Die erste Zeitspanne umfasst vorzugsweise das Anfluten des Untersuchungsbereichs mit dem Kontrastmittel. Vorzugsweise umfasst die erste Zeitspanne die arterielle Phase und/oder die portalvenöse Phase und/oder die Spätphase bei der dynamischen kontrastverstärkenden Magnetresonanztomographie einer Leber oder eines Teils einer Leber eines Untersuchungsobjekts. Die genannten Phasen sind beispielsweise in den folgenden Publikationen definiert und beschrieben: Abdominal Imaging, 2015, Vol. 40, 64-75, DOI: 10.1007/S00261-014-0188-8, J.

Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

In einer bevorzugten Ausführungsform wird die erste Zeitspanne so gewählt, dass solche MRT-Aufnahmen von der Leber oder einem Teil der Leber eines Untersuchungsobjekts erzeugt werden,
(i) die den Untersuchungsbereich ohne Kontrastmittel zeigen,
(ii) die den Untersuchungsbereich während der arteriellen Phase zeigen, in der das Kontrastmittel sich über die Arterien in dem Untersuchungsbereich ausbreitet,
(iii) die den Untersuchungsbereich während der portalvenösen Phase zeigen, in der das Kontrastmittel über die Pfortader in den Untersuchungsbereich gelangt, und
(iv) die den Untersuchungsbereich während der Spätphase zeigen, in der die Konzentration des Kontrastmittels in den Arterien und Venen zurückgeht und die Konzentration des Kontrastmittels in den Leberzellen ansteigt.

Erfindungsgemäß beginnt die erste Zeitspanne in einer Zeitspanne von einer Minute bis einer Sekunde vor der Applikation des Kontrastmittels, oder mit der Applikation des Kontrastmittels, und dauert ab der Applikation des Kontrastmittels eine Zeitspanne von 2 Minuten bis 15 Minuten, vorzugsweise 2 Minuten bis 13 Minuten, noch mehr bevorzugt 3 Minuten bis 10 Minuten. Da das Kontrastmittel renal und biliär sehr langsam ausgeschieden wird, kann sich die zweite Zeitspanne bis zu zwei Stunden und mehr nach der Applikation des Kontrastmittels hinziehen.

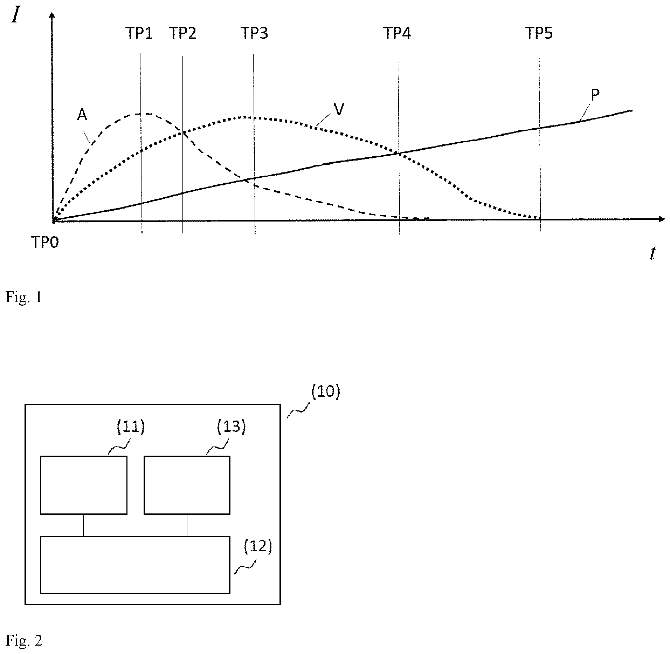

Da sich Kontrastmittel in verschiedenen Untersuchungsobjekten unterschiedlich schnell ausbreiten kann, kann die erste Zeitspanne auch über die Konzentrationen des Kontrastmittels in den unterschiedlichen Arealen des Untersuchungsbereichs definiert werden. Eine Möglichkeit ist in Figur 1 dargestellt. Figur 1 zeigt schematisch den zeitlichen Verlauf der Konzentrationen von Kontrastmittel in den Leberarterien (A), den Lebervenen (V) und den gesunden Leberzellen (P). Die Konzentrationen sind in Form der Signalintensitäten *I* in den genannten Arealen (Leberarterien, Lebervenen, Leberzellen) bei der Magnetresonanzmessung als Funktion der Zeit t dargestellt. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (V) steigt langsamer an als in den Leberarterien und erreicht ihr Maximum später (gepunktete Kurve). Die Konzentration des Kontrastmittels in den gesunden Leberzellen (P) steigt langsam an (durchgezogene Kurve) und erreicht ihr Maximum erst zu einem sehr viel späteren Zeitpunkt (das Maximum ist in der Figur 1 nicht dargestellt). Es lassen sich einige charakteristische Zeitpunkte definieren: Zum Zeitpunkt TP0 wird Kontrastmittel intravenös als Bolus appliziert. Zum Zeitpunkt TP1 erreicht die Konzentration (die Signalintensität) des Kontrastmittels in den Leberarterien ihr Maximum. Zum Zeitpunkt TP2 schneiden sich die Kurven der Signalintensitäten bei den Leberarterien und den Lebervenen. Zum Zeitpunkt TP3 durchläuft die Konzentration (die Signalintensität) des Kontrastmittels in den Lebervenen ihr Maximum. Zum Zeitpunkt TP4 schneiden sich die Kurven der Signalintensitäten bei den Leberarterien und den Leberzellen. Zum Zeitpunkt T5 sind die Konzentrationen in den Leberarterien und den Lebervenen auf ein Niveau gesunken, bei dem sie keine messbare Kontrastverstärkung mehr verursachen.

In einer bevorzugten Ausführungsform umfasst die erste Zeitspanne zumindest die Zeitpunkte TP0, TP1, TP2, TP3 und TP4.

In einer bevorzugten Ausführungsform werden mindestens MRT-Aufnahmen von allen folgenden Phasen (messtechnisch) erzeugt: in einer Zeitspanne vor TP0, in der Zeitspanne von TP0 bis TP1, in der Zeitspanne von TP1 bis TP2, in der Zeitspanne von TP2 bis TP3 und in der Zeitspanne TP3 bis TP4.

Es ist denkbar, dass in den Zeitspannen vor TP0, von TP0 bis TP1, von TP1 bis TP2, von TP2 bis TP3 und von TP3 bis TP4 jeweils eine oder mehrere MRT-Aufnahmen (messtechnisch) erzeugt werden. Denkbar ist auch, dass während einer oder mehrerer Zeitspannen Sequenzen von MRT-Aufnahmen (messtechnisch) erzeugt werden.

Der Begriff Sequenz bedeutet zeitliche Abfolge, d.h. es werden mehrere MRT-Aufnahmen erzeugt, die den Untersuchungsbereich zu nacheinander folgenden Zeitpunkten zeigen.

Jeder MRT-Aufnahme ist ein Zeitpunkt zugeordnet oder jeder MRT-Aufnahme lässt sich ein Zeitpunkt zuordnen. Üblicherweise handelt es sich bei diesem Zeitpunkt um den Zeitpunkt, an dem die MRT-Aufnahme erzeugt worden ist (absolute Zeit). Dem Fachmann ist bewusst, dass die Erzeugung einer MRT-Aufnahme eine gewisse Zeitspanne in Anspruch nimmt. Einer MRT-Aufnahme kann z.B. der Zeitpunkt des Beginns der Aufnahme oder der Zeitpunkt der Fertigstellung der Aufnahme zugeordnet werden. Es ist aber auch denkbar, dass den MRT-Aufnahmen willkürliche Zeitpunkte zugeordnet werden (z.B. relative Zeitpunkte).

Anhand eines Zeitpunktes lässt sich eine MRT-Aufnahme zeitlich in Bezug auf eine andere MRT-Aufnahme einordnen; anhand des Zeitpunkts einer MRT-Aufnahme lässt sich feststellen, ob der in der MRT-Aufnahme gezeigte Moment zeitlich vor oder zeitlich nach einem, in einer anderen MRT-Aufnahme gezeigten Moment stattgefunden hat.

Vorzugsweise sind die MRT-Aufnahmen in einer Sequenz und einer Mehrzahl zeitlich so geordnet, dass MRT-Aufnahmen, die einen früheren Zustand des Untersuchungsbereichs zeigen, in der Sequenz bzw. der Mehrzahl vor solchen MRT-Aufnahmen angeordnet sind, die einen späteren Zustand des Untersuchungsbereichs zeigen.

Die Zeitspanne zwischen zwei in einer Sequenz und/oder Mehrzahl unmittelbar aufeinander folgenden MRT-Aufnahmen ist vorzugsweise für alle Paare von in der Sequenz und/oder Mehrzahl unmittelbar aufeinander folgenden MRT-Aufnahmen gleich, d.h. die MRT-Aufnahmen wurden vorzugsweise mit einer konstanten Aufnahmerate erzeugt.

Auf Basis der (messtechnisch) während der ersten Zeitspanne erzeugten (ersten) MRT-Aufnahmen wird eine zweite MRT-Aufnahme oder werden mehrere zweite MRT-Aufnahmen, die den Untersuchungsbereich während einer zweiten Zeitspanne zeigen, vorhergesagt. MRT-Aufnahmen, die für die zweite Zeitspanne vorhergesagt werden, werden in dieser Beschreibung auch als zweite MRT-Aufnahmen bezeichnet.

In einer bevorzugten Ausführungsform der vorliegenden Erfindung schließt sich die zweite Zeitspanne an die erste Zeitspanne an.

Bei der zweiten Zeitspanne handelt es sich vorzugsweise um eine Zeitspanne innerhalb der hepatobiliären Phase; erfindungsgemäß handelt es sich um eine Zeitspanne, die mindestens 10 Minuten nach Applikation des Kontrastmittels einsetzt, vorzugsweise mindestens 20 Minuten nach Applikation des Kontrastmittels.

Die Mehrzahl der gemessenen MRT-Aufnahmen, die den Untersuchungsbereich während der ersten Zeitspanne zeigen, wird einem Vorhersagemodell zugeführt. Das Vorhersagemodell ist ein Modell, das konfiguriert ist, auf Basis einer Mehrzahl an MRT-Aufnahmen, die einen Untersuchungsbereich während einer ersten Zeitspanne zeigen, eine oder mehrere MRT-Aufnahmen vorherzusagen, die den Untersuchungsbereich während einer zweiten Zeitspanne zeigen.

Dabei bedeutet der Begriff "Vorhersage", dass die MRT-Aufnahmen, die den Untersuchungsbereich während der zweiten Zeitspanne zeigen, unter Verwendung der MRT-Aufnahmen, die den Untersuchungsbereich während der ersten Zeitspanne zeigen, berechnet werden.

Das Vorhersagemodell wurde erfindungsgemäß mit Hilfe eines selbstlernenden Algorithmus in einem überwachten maschinellen Lernen erstellt. Zum Lernen werden Trainingsdaten verwendet, die eine Vielzahl an MRT-Aufnahmen der ersten und der zweiten Zeitspanne umfassen. Diese Trainingsdaten werden in dieser Beschreibung auch als Referenz-MRT-Aufnahmen bezeichnet. Referenz-MRT-Aufnahmen, die während der ersten Zeitspanne erzeugt worden sind, werden auch als erste Referenz-MRT-Aufnahmen bezeichnet; Referenz-MRT-Aufnahmen, die während der zweiten Zeitspanne erzeugt worden sind, werden auch als zweite Referenz-MRT-Aufnahmen bezeichnet.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann das Vorhersagemodell auch unbekannte Daten beurteilen. Validierungsdaten können verwendet werden, um die Güte der Beurteilung unbekannter Daten zu prüfen.

Das Trainieren des Vorhersagemodells erfolgt mittels überwachten Lernens (engl.: *supervised learning*)*,* d.h. dem Algorithmus werden nacheinander Mehrzahlen von ersten Referenz-MRT-Aufnahmen aus der ersten Zeitspanne präsentiert und es wird ihm "mitgeteilt", welche zweiten Referenz-MRT-Aufnahmen in der zweiten Zeitspanne mit diesen Mehrzahlen verbunden sind. Der Algorithmus lernt dann eine Beziehung zwischen den Mehrzahlen der Referenz-MRT-Aufnahmen der ersten Zeitspanne und den Referenz-MRT-Aufnahmen der zweiten Zeitspanne, um für unbekannte Mehrzahlen von MRT-Aufnahmen der ersten Zeitspanne eine oder mehrere MRT-Aufnahmen in der zweiten Zeitspanne vorherzusagen.

Selbstlernende Systeme, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt, zu Bedingungen, die bei der Erzeugung der MRT-Aufnahmen herrschten, und/oder Informationen zu den Zeitpunkten oder Zeitspannen zu/in denen die MRT-Aufnahmen erzeugt worden sind) vorhanden sein.

In einem solchen Netzwerk dienen die Ausgangsneuronen dazu, für eine Mehrzahl von MRT-Aufnahmen einer ersten Zeitspanne eine oder mehrere MRT-Aufnahmen einer zweiten Zeitspanne vorherzusagen.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufhahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Bei der Analyse von Sequenzen (Abfolgen von MRT-Aufnahme) können Raum und Zeit als äquivalente Dimensionen behandelt und z.B. über 3D-Faltungen verarbeitet werden. Dies wurde in den Arbeiten von Baccouche et al. gezeigt (siehe z.B. Baccouche et al.: Sequential Deep Learning for Human Action Recognition; International Workshop on Human Behavior Understanding, Springer 2011, pages 29-39) und Ji et al. (3D Convolutional Neural Networks for Human Action Recognition, IEEE Transactions on Pattern Analysis and Machine Intelligence, 35(1), 221-231).

Ferner kann man verschiedene Netzwerke trainieren, die für Zeit und Raum verantwortlich sind, und schließlich die Merkmale verschmelzen, wie in Veröffentlichungen von Karpathy et al. und Simonyan & Zisserman beschrieben ist (siehe z.B. Karpathy et al.: Large-scale Video Classification with Convolutional Neural Networks; Proceedings of the IEEE conference on Computer Vision and Pattern Recognition, 2014, pages 1725-1732; Simonyan & Zisserman: Two-stream Convolutional Networks for Action Recognition in Videos; Advances in Neural Information Processing Systems, 2014, pages 568-576).

Rekurrente neuronale Netze (RNNs) sind eine Familie von Neuronalen Netzen, die Rückkopplungsverbindungen zwischen Schichten enthalten. RNNs ermöglichen die Modellierung sequentieller Daten durch gemeinsame Nutzung von Parameterdaten über verschiedene Teile des neuronalen Netzwerks. Die Architektur für ein RNN enthält Zyklen. Die Zyklen repräsentieren den Einfluss eines gegenwärtigen Werts einer Variablen auf ihren eigenen Wert zu einem zukünftigen Zeitpunkt, da mindestens ein Teil der Ausgabedaten von der RNN als Rückkopplung zur Verarbeitung nachfolgender Eingaben in einer Sequenz verwendet wird.

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226, WO2018/183044A1, WO2018/200493, WO2019/074938A1, WO2019/204406A1, WO2019/241659A1).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabedaten auf gegebene Ausgabedaten angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte .

Im trainierten Zustand enthalten die Verbindungsgewichte zwischen den Verarbeitungselementen Informationen bezüglich der Beziehung zwischen den Mehrzahlen der Referenz-MRT-Aufnahmen der ersten Zeitspanne und den Referenz-MRT-Aufnahmen der zweiten Zeitspanne, die verwendet werden können, um eine oder mehrere MRT-Aufnahmen, die einen Untersuchungsbereich während der zweiten Zeitspanne zeigen, für neue Mehrzahlen von MRT-Aufnahmen, die den Untersuchungsbereich während der ersten Zeitspanne zeigen, vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Mehrzahlen von MRT-Aufnahmen anwenden lässt.

Wie bereits beschrieben, können zum Trainieren, Validieren und Vorhersagen auch weitere Informationen zum Untersuchungsobjekt, zum Untersuchungsbereich, zu Untersuchungsbedingungen und/oder zu den gemessenen MRT-Aufnahmen verwendet werden.

Beispiele für Informationen zum Untersuchungsobjekt sind: Geschlecht, Alter, Gewicht, Größe, Anamnese, Art und Dauer und Menge bereits eingenommener Medikamente, Blutdruck, zentralvenöser Druck, Atemfrequenz, Serum Albumin, Total Bilirubin, Blutzucker, Eisengehalt, Atemkapazität und dergleichen. Diese können z.B. auch einer Datenbank bzw. einer elektronischen Patientenakte herangezogen werden.

Beispiele für Informationen zum Untersuchungsbereich sind: Vorerkrankungen, Operationen, Teilresektion, Lebertransplantation, Eisenleber, Fettleber und dergleichen.

Es ist denkbar, dass die Mehrzahl an MRT-Aufnahmen, die den Untersuchungsbereich während der ersten Zeitspanne zeigen, einer Bewegungskorrektur unterzogen werden, bevor sie dem Vorhersagemodell zugeführt werden. Eine solche Bewegungskorrektur sorgt dafür, dass ein Pixel oder Voxel einer ersten MRT-Aufnahme denselben Untersuchungsbereich zeigt, wie das entsprechende Pixel oder Voxel einer zweiten, zeitlich nachgelagerten MRT-Aufnahme. Bewegungskorrekturverfahren sind im Stand der Technik beschrieben (siehe zum Beispiel: EP3118644, EP3322997, US20080317315, US20170269182, US20140062481, EP2626718).

Ein Gegenstand der vorliegenden Erfindung ist ein System, mit dem das erfindungsgemäße Verfahren ausgeführt werden kann.

Das System umfasst eine Empfangseinheit, eine Steuer- und Recheneinheit und eine Ausgabeeinheit.

Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon und/oder dergleichen.

Das erfindungsgemäße System ist konfiguriert, Mehrzahlen von MRT-Aufnahmen, die einen Untersuchungsbereich während einer ersten Zeitspanne zeigen, zu empfangen und auf Basis dieser Daten und ggf. weiterer Daten eine oder mehrere MRT-Aufnahmen zu erzeugen (vorherzusagen, zu berechnen), die den Untersuchungsbereich während einer zweiten Zeitspanne zeigen.

Die Steuer- und Recheneinheit dient der Steuerung der Empfangseinheit, der Koordinierung der Daten- und Signalflüsse zwischen verschiedenen Einheiten und der Berechnung von MRT-Aufnahmen. Es ist denkbar, dass mehrere Steuer- und Recheneinheiten vorhanden sind.

Die Empfangseinheit dient zum Empfang von Mehrzahlen von MRT-Aufnahmen. Die Mehrzahlen können beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Von der Empfangseinheit werden die Sequenzen der MRT-Aufnahmen und ggf. weitere Daten an die Steuer- und Recheneinheit übermittelt.

Die Steuer- und Recheneinheit ist konfiguriert, anhand der Mehrzahlen der MRT-Aufnahmen, die einen Untersuchungsbereich während einer ersten Zeitspanne zeigen, eine oder mehrere MRT-Aufnahmen vorherzusagen, wobei die vorhergesagten MRT-Aufnahmen den Untersuchungsbereich während einer zweiten Zeitspanne zeigen. Vorzugsweis kann in einen Arbeitsspeicher der Steuer- und Recheneinheit ein Vorhersagemodell geladen werden, mit dem die MRT-Aufnahmen der zweiten Zeitspanne berechnet werden. Das Vorhersagemodell wurde erfindungsgemäß mit Hilfe eines selbstlernenden Algorithmus mittels überwachtem Lernen erzeugt (trainiert).

Über die Ausgabeeinheit können die vorgesagten MRT-Aufhahmen angezeigt werden (zum Beispiel auf einem Bildschirm), ausgegeben werden (z.B. über einen Drucker) oder in einem Datenspeicher gespeichert werden.

Die Erfindung wird nachstehend anhand von Figuren näher erläutert, ohne die Erfindung auf die in den Figuren gezeigten Merkmale oder Merkmalskombinationen beschränken zu wollen.

Es zeigen:
Figur 1 zeigt schematisch den zeitlichen Verlauf der Konzentrationen von Kontrastmittel in den Leberarterien (A), den Lebervenen (V) und den Leberzellen (P) und ist bereits oben detailliert beschrieben worden.
Figur 2 zeigt schematisch eine bevorzugte Ausführungsform des erfindungsgemäßen Systems. Das System (10) umfasst eine Empfangseinheit (11), eine Steuer- und Recheneinheit (12) und eine Ausgabeeinheit (13).

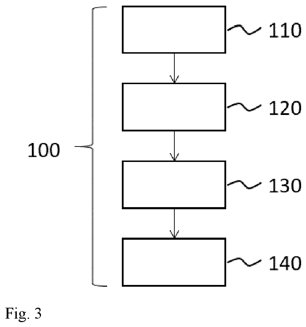

Figur 3 zeigt schematisch und beispielhaft eine Ausführungsform des erfindungsgemäßen Verfahrens.

Das Verfahren (100) umfasst die Schritte:
(110) Empfangen einer Mehrzahl von ersten MRT-Aufnahmen, wobei zumindest ein Teil der ersten MRT-Aufnahmen einen Untersuchungsbereich während einer ersten Zeitspanne nach einer Applikation eines Kontrastmittels zeigt,
(120) Zuführen der Mehrzahl von ersten MRT-Aufnahmen einem Vorhersagemodell, wobei das Vorhersagemodell anhand von Referenz-MRT-Aufnahmen mittels überwachten Lernens trainiert worden ist, aus ersten Referenz-MRT-Aufnahmen, die einen Untersuchungsbereich während einer ersten Zeitspanne zeigen, eine oder mehrere zweite Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
(130) Erzeugen einer oder mehrerer vorhergesagter MRT-Aufnahmen, die den Untersuchungsbereich während einer zweiten Zeitspanne zeigen, durch das Vorhersagemodell, wobei die zweite Zeitspanne zeitlich auf die erste Zeitspanne folgt,
(140) Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

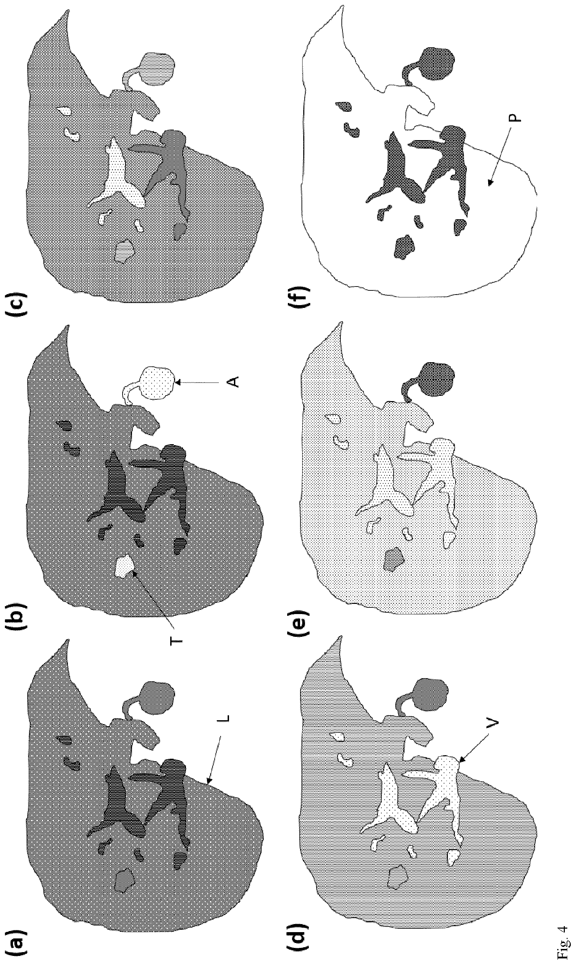

Figur 4 zeigt schematisch und beispielhaft eine Mehrzahl an MRT-Aufnahmen der Leber während der dynamischen und der hepatobiliären Phase. In den Figuren 4 (a), 4 (b), 4 (c), 4 (d), 4 (e) und 4 (f) ist stets derselbe Querschnitt durch die Leber zu unterschiedlichen Zeitpunkten dargestellt. Die in den Figuren 4 (a), 4 (b), 4 (d) und 4 (f) eingezeichneten Bezugszeichen gelten für alle Figuren 4 (a), 4 (b), 4 (c), 4 (d), 4 (e) und 4 (f); sie sind lediglich der besseren Übersicht halber jeweils nur einmal eingezeichnet.

Fig. 4 (a) zeigt den Querschnitt durch die Leber (L) vor der intravenösen Applikation eines hepatobiliären Kontrastmittels. Zu einem Zeitpunkt, der zwischen den Zeitpunkten liegt, die durch die Figuren 4 (a) und 4 (b) dargestellt werden, wurde ein hepatobiliäres Kontrastmittel intravenös als Bolus verabreicht. Dieses erreicht in Fig. 4 (b) über die Leberarterie (A) die Leber. Dementsprechend wird die Leberarterie signalverstärkt dargestellt (arterielle Phase). Ein Tumor (T), der hauptsächlich über Arterien mit Blut versorgt wird, hebt sich ebenfalls als hellerer (signalverstärkter) Bereich vor dem Leberzellengewebe ab. Zu dem Zeitpunkt, der in Figur 4 (c) dargestellt ist, erreicht das Kontrastmittel die Leber über die Venen. In Figur 4 (d) heben sich die venösen Blutgefäße (V) als helle (signalverstärkte) Bereiche von dem Lebergewebe ab (venöse Phase). Gleichzeitig steigt die Signalintensität in den gesunden Leberzellen, die hauptsächlich über die Venen mit Kontrastmittel versorgt werden, kontinuierlich an (Fig. 4 (c) ---> 4 (d) ---> 4 (e) ---> 4 (f)). In der hepatobiliären Phase, die in Fig. 4 (f) dargestellt ist, sind die Leberzellen (P) signalverstärkt dargestellt; die Blutgefäße und der Tumor weisen kein Kontrastmittel mehr auf und sind entsprechend dunkel dargestellt.

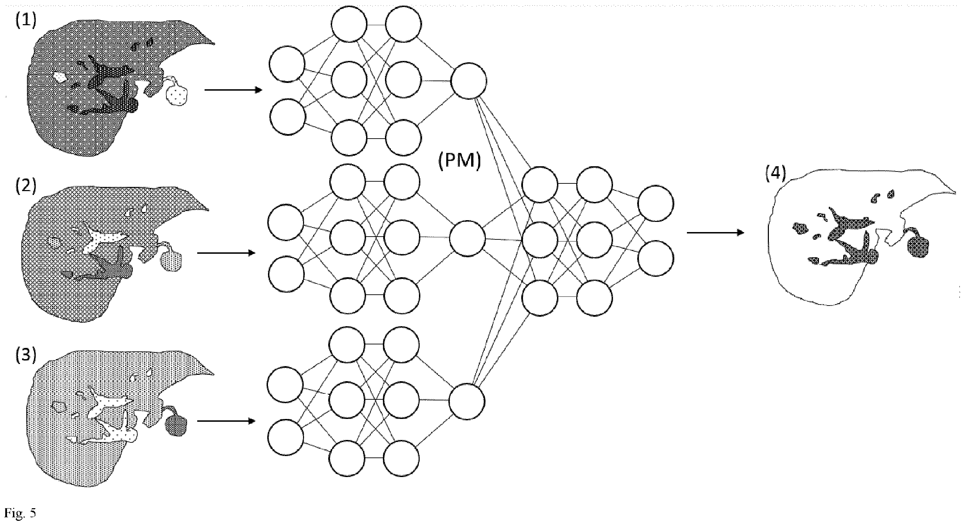

Fig. 5 zeigt beispielhaft und schematisch, wie drei MRT-Aufnahmen (1), (2) und (3), die eine Leber in einer ersten Zeitspanne zeigen, einem Vorhersagemodell (PM) zugeführt werden. Das Vorhersagemodell berechnet aus den drei MRT-Aufnahmen (1), (2) und (3) eine MRT-Aufnahme (4), die die Leber in einer zweiten Zeitspanne zeigt. Bei den MRT-Aufnahmen (1), (2) und (3) kann es sich beispielsweise um die in Fig. 4 (b), 4 (c) und 4 (d) gezeigten MRT-Aufnahmen zeigen, Bei der MRT-Aufnahme (4) kann es sich beispielsweise um die in Fig. 4 (f) gezeigte MRT-Aufnahme handeln.

## Patentansprüche

1. Computer-implementiertes Verfahren umfassend die Schritte
- Empfangen einer Mehrzahl von MRT-Aufnahmen, wobei zumindest ein Teil der MRT-Aufnahmen einen Untersuchungsbereich während einer ersten Zeitspanne nach einer Applikation eines Kontrastmittels zeigt,
- Zuführen der Mehrzahl von MRT-Aufnahmen (1, 2, 3) einem Vorhersagemodell (PM),
- Erzeugen einer oder mehrerer vorhergesagter MRT-Aufnahmen (4), die den Untersuchungsbereich während einer zweiten Zeitspanne zeigen, durch das Vorhersagemodell (PM), wobei die zweite Zeitspanne zeitlich auf die erste Zeitspanne folgt,
- Anzeigen und/oder Ausgeben der einen oder der mehreren vorhergesagten MRT-Aufnahmen (4) und/oder Speichern der einen oder der mehreren vorhergesagten MRT-Aufnahmen (4) in einem Datenspeicher,
wobei das Vorhersagemodell (PM) mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
wobei der Untersuchungsbereich eine Leber oder ein Teil einer Leber eines Menschen ist,
wobei das Kontrastmittel ein hepatobiliäres Kontrastmittel ist,
wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert,
wobei die zweite Zeitspanne mindestens 10 Minuten nach der Applikation des Kontrastmittels beginnt.

2. Verfahren gemäß Anspruch 1, wobei mindestens eine der empfangenen MRT-Aufnahmen (1, 2, 3) den Untersuchungsbereich vor der Applikation des Kontrastmittels zeigt und mindestens eine der empfangenen MRT-Aufnahmen (1, 2, 3) den Untersuchungsbereich nach der Applikation des Kontrastmittels zeigt.

3. Verfahren gemäß Anspruch 2, wobei die erste Zeitspanne so gewählt ist, dass sie den Untersuchungsbereich in unterschiedlichen Phasen zeigt, wobei die Phasen eine native Phase, eine arterielle Phase, eine portalvenöse Phase und eine Spätphase umfassen, wobei mindestens eine MRT-Aufnahme empfangen wird, die den Untersuchungsbereich in der nativen Phase zeigt, und mindestens eine MRT-Aufnahme empfangen wird, die den Untersuchungsbereich während der arteriellen Phase zeigt, und mindestens eine MRT-Aufnahme empfangen wird, die den Untersuchungsbereich in der portalvenösen Phase zeigt, und mindestens eine MRT-Aufnahme empfangen wird, die den Untersuchungsbereich in der Spätphase zeigt.

4. Verfahren gemäß einem der Ansprüche 1 bis 3, wobei die empfangenen MRT-Aufnahmen (1, 2, 3) eine Leber oder einen Teil einer Leber eines Säugetiers vor einem Zeitpunkt TP0 und während einer Zeitspanne von TP0 bis TP1 und/oder während einer Zeitspanne von TP1 bis TP2 und/oder während einer Zeitspanne von TP2 bis TP3 und/oder während einer Zeitspanne von TP3 bis TP4 zeigen, wobei zum Zeitpunkt TP0 das Kontrastmittel intravenös als Bolus appliziert wird und dann über Leberarterien und Lebervenen in Leberzellen gelangt, wobei zum Zeitpunkt TP1 das Kontrastmittel in den Leberarterien eine maximale Konzentration erreicht, wobei zum Zeitpunkt TP2 eine durch das Kontrastmittel in den Lebervenen erzeugte Signalintensität einen Wert annimmt, der so groß ist wie ein Wert einer durch das Kontrastmittel in den Leberarterien erzeugten Signalintensität, wobei zum Zeitpunkt TP3 das Kontrastmittel in den Lebervenen eine maximale Konzentration erreicht, wobei zum Zeitpunkt TP4 eine durch das Kontrastmittel in den Leberzellen erzeugte Signalintensität einen Wert annimmt, der so groß ist wie ein Wert einer durch das Kontrastmittel in den Lebervenen erzeugten Signalintensität.

5. Verfahren gemäß einem der Ansprüche 1 bis 4, wobei die erste Zeitspanne ab der Applikation des Kontrastmittels eine Zeitspanne 2 Minuten bis 13 Minuten, bevorzugt 3 Minuten bis 10 Minuten andauert.

6. Verfahren gemäß einem der Ansprüche 1 bis 5, wobei die zweite Zeitspanne innerhalb einer hepatobiliären Phase liegt.

7. Verfahren gemäß einem der Ansprüche 1 bis 6, wobei die zweite Zeitspanne mindestens 20 Minuten nach der Applikation des Kontrastmittels beginnt.

8. Verfahren gemäß einem der Ansprüche 1 bis 7, wobei es sich bei dem Vorhersagemodell (PM) um ein künstliches neuronales Netz handelt.

9. Verfahren gemäß einem der Ansprüche 1 bis 8, wobei es sich bei dem Kontrastmittel um Gd-EOB-DTPA oder Gd-BOPTA handelt.

10. System (10) umfassend
• eine Empfangseinheit (11),
• eine Steuer- und Recheneinheit (12) und
• eine Ausgabeeinheit (13),
- wobei die Steuer- und Recheneinheit (12) konfiguriert ist, die Empfangseinheit (11) zu veranlassen, eine Mehrzahl von MRT-Aufnahmen (1, 2, 3) zu empfangen, wobei zumindest ein Teil der MRT-Aufnahmen (1, 2, 3) einen Untersuchungsbereich während einer ersten Zeitspanne nach einer Applikation eines Kontrastmittels zeigt,
- wobei die Steuer- und Recheneinheit (12) konfiguriert ist, anhand der empfangenen MRT-Aufnahmen (1, 2, 3) eine oder mehrere MRT-Aufnahmen (4) mittels eines Vorhersagemodells vorherzusagen, wobei die eine oder die mehreren vorhergesagten MRT-Aufnahmen (4) den Untersuchungsbereich während einer zweiten Zeitspanne zeigen, wobei die zweite Zeitspanne zeitlich auf die erste Zeitspanne folgt,
- wobei die Steuer- und Recheneinheit (12) konfiguriert ist, die Ausgabeeinheit (13) zu veranlassen, die eine oder die mehreren vorhergesagten MRT-Aufnahmen (4) anzuzeigen, auszugeben oder in einem Datenspeicher zu speichern,
wobei das Vorhersagemodell (PM) mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
wobei der Untersuchungsbereich eine Leber oder ein Teil einer Leber eines Menschen ist,
wobei das Kontrastmittel ein hepatobiliäres Kontrastmittel ist,
wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert,
wobei die zweite Zeitspanne mindestens 10 Minuten nach der Applikation des Kontrastmittels beginnt.

11. Computerprogrammprodukt umfassend ein Computerprogramm, das in einen Arbeitsspeicher eines Computersystems geladen werden kann und dort das Computersystem dazu veranlasst, folgende Schritte ausfuhren:
- Empfangen einer Mehrzahl von MRT-Aufnahmen (1, 2, 3), wobei zumindest ein Teil der MRT-Aufnahmen (1, 2, 3) einen Untersuchungsbereich während einer ersten Zeitspanne nach einer Applikation eines Kontrastmittels zeigt,
- Zuführen der Mehrzahl von MRT-Aufnahmen einem Vorhersagemodell (PM),
- Erzeugen einer oder mehrerer vorhergesagter MRT-Aufnahmen (4), die den Untersuchungsbereich während einer zweiten Zeitspanne zeigen, durch das Vorhersagemodell (PM), wobei die zweite Zeitspanne zeitlich auf die erste Zeitspanne folgt,
- Anzeigen und/oder Ausgeben der einen oder der mehreren vorhergesagten MRT-Aufnahmen (4) und/oder Speichern der einen oder der mehreren vorhergesagten MRT-Aufnahmen (4) in einem Datenspeicher,
wobei das Vorhersagemodell (PM) mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
wobei der Untersuchungsbereich eine Leber oder ein Teil einer Leber eines Menschen ist,
wobei das Kontrastmittel ein hepatobiliäres Kontrastmittel ist,
wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert,
wobei die zweite Zeitspanne mindestens 10 Minuten nach der Applikation des Kontrastmittels beginnt.

12. Verwendung eines Kontrastmittels in einem Computer-implementierten MRT-Verfahren, nachdem ein Kontrastmittel appliziert wurde und sich in einem Untersuchungsbereich verteilt hat, wobei das Computer-implementierte MRT-Verfahren die folgenden Schritte umfasst:
- Erzeugen einer Mehrzahl von MRT-Aufnahmen (1, 2, 3) von dem Untersuchungsbereich während einer ersten Zeitspanne
- Zuführen der erzeugten MRT-Aufnahmen (1, 2, 3) einem Vorhersagemodell (PM),
- Empfangen einer oder mehrerer vorhergesagter MRT-Aufnahmen (4), die den Untersuchungsbereich während einer zweiten Zeitspanne zeigen, als Ausgabe von dem Vorhersagemodel (PM), wobei die zweite Zeitspanne zeitlich auf die erste Zeitspanne folgt,
- Anzeigen und/oder Ausgeben der einen oder der mehreren vorhergesagten MRT-Aufnahmen (4) und/oder Speichern der einen oder der mehreren vorhergesagten MRT-Aufnahmen (4) in einem Datenspeicher,
wobei das Vorhersagemodell (PM) mittels überwachten Lernens trainiert worden ist, anhand von Referenz-MRT-Aufnahmen, von denen zumindest ein Teil einen Untersuchungsbereich während einer ersten Zeitspanne nach einer Applikation eines Kontrastmittels zeigt, eine oder mehrere Referenz-MRT-Aufnahmen vorherzusagen, die den Untersuchungsbereich während einer zweiten Zeitspanne zeigen, wobei die zweite Zeitspanne zeitlich auf die erste Zeitspanne folgt,
wobei der Untersuchungsbereich eine Leber oder ein Teil einer Leber eines Menschen ist,
wobei das Kontrastmittel ein hepatobiliäres Kontrastmittel ist,
wobei die erste Zeitspanne in einer Zeitspanne von einer Minute bis einer Sekunde vor der Applikation des Kontrastmittels oder mit der Applikation des Kontrastmittels beginnt, und ab der Applikation des Kontrastmittels eine Zeitspanne von 2 Minuten bis 15 Minuten andauert,
wobei die zweite Zeitspanne mindestens 10 Minuten nach der Applikation des Kontrastmittels beginnt.

13. Kit umfassend ein Kontrastmittel und ein Computerprogrammprodukt gemäß Anspruch 11, wobei das Kontrastmittel ein hepatobiliäres MRT-Kontrastmittel ist.

## Claims

1. Computer-implemented method comprising the steps of
- receiving a plurality of MRI images, at least some of the MRI images showing an examination region during a first time span after administering a contrast agent,
- feeding the plurality of MRI images (1, 2, 3) to a prediction model (PM),
- generating one or more predicted MRI images (4) showing the examination region during a second time span by means of the prediction model (PM), the second time span following the first time span chronologically,
- displaying and/or outputting the one or more predicted MRI images (4) and/or storing the one or more predicted MRI images (4) in a data storage medium,
the prediction model (PM) having been trained by means of supervised learning to predict, on the basis of reference MRI images, at least some of which show an examination region during a first time span after administering a contrast agent, one or more reference MRI images showing the examination region during a second time span, the second time span following the first time span chronologically,
the examination region being a liver or a portion of a liver of a human,
the contrast agent being a hepatobiliary contrast agent,
the first time span starting within a time span of from one minute to one second before the administration of the contrast agent or with the administration of the contrast agent, and lasting for a time span of from 2 minutes to 15 minutes from the administration of the contrast agent, the second time span starting at least 10 minutes after the administration of the contrast agent.

2. Method according to Claim 1, wherein at least one of the received MRI images (1, 2, 3) shows the examination region before administering the contrast agent and at least one of the received MRI images (1, 2, 3) shows the examination region after administering the contrast agent.

3. Method according to Claim 2, wherein the first time span is chosen such that it shows the examination region in different phases, wherein the phases comprise a native phase, an arterial phase, a portal-vein phase and a late phase, wherein at least one MRI image is received which shows the examination region in the native phase, and at least one MRI image is received which shows the examination region during the arterial phase, and at least one MRI image is received which shows the examination region in the portal-vein phase, and at least one MRI image is received which shows the examination region in the late phase.

4. Method according to any of Claims 1 to 3, wherein the received MRI images (1, 2, 3) show a liver or a portion of a liver of a mammal prior to a time point TP0 and during a time span from TP0 to TP1 and/or during a time span from TP1 to TP2 and/or during a time span from TP2 to TP3 and/or during a time span from TP3 to TP4, at time point TP0 the contrast agent being administered intravenously as a bolus and then reaching liver cells via liver arteries and liver veins, at time point TP1 the contrast agent in the liver arteries reaching a maximum concentration, at time point TP2 a signal intensity generated in the liver veins by the contrast agent assuming a value which is the same size as a value of a signal intensity generated in the liver arteries by the contrast agent, at time point TP3 the contrast agent in the liver veins reaching a maximum concentration, at time point TP4 a signal intensity generated in the liver cells by the contrast agent assuming a value which is the same size as a value of a signal intensity generated in the liver veins by the contrast agent.

5. Method according to any of Claims 1 to 4, wherein the first time span lasts for a time span of from 2 minutes to 13 minutes, preferably 3 minutes to 10 minutes, from the administration of the contrast agent.

6. Method according to any of Claims 1 to 5, wherein the second time span is within a hepatobiliary phase.

7. Method according to any of Claims 1 to 6, wherein the second time span starts at least 20 minutes after the administration of the contrast agent.

8. Method according to any of Claims 1 to 7, wherein the prediction model (PM) is an artificial neural network.

9. Method according to any of Claims 1 to 8, wherein the contrast agent is Gd-EOB-DTPA or Gd-BOPTA.

10. System (10) comprising
• a receiving unit (11),
• a control and calculation unit (12) and
• an output unit (13),
- the control and calculation unit (12) being configured to prompt the receiving unit (11) to receive a plurality of MRI images (1, 2, 3), at least some of the MRI images (1, 2, 3) showing an examination region during a first time span after administering a contrast agent,
- the control and calculation unit (12) being configured to predict one or more MRI images (4) by means of a prediction model on the basis of the received MRI images (1, 2, 3), the one or more predicted MRI images (4) showing the examination region during a second time span, the second time span following the first time span chronologically,
- the control and calculation unit (12) being configured to prompt the output unit (13) to display the one or more predicted MRI images (4), to output them or to store them in a data storage medium,
the prediction model (PM) having been trained by means of supervised learning to predict, on the basis of reference MRI images, at least some of which show an examination region during a first time span after administering a contrast agent, one or more reference MRI images showing the examination region during a second time span, the second time span following the first time span chronologically,
the examination region being a liver or a portion of a liver of a human,
the contrast agent being a hepatobiliary contrast agent, the first time span starting within a time span of from one minute to one second before the administration of the contrast agent or with the administration of the contrast agent, and lasting for a time span of from 2 minutes to 15 minutes from the administration of the contrast agent, the second time span starting at least 10 minutes after the administration of the contrast agent.

11. Computer program product comprising a computer program which can be loaded into a memory of a computer system, where it prompts the computer system to execute the following steps:
- receiving a plurality of MRI images (1, 2, 3), at least some of the MRI images (1, 2, 3) showing an examination region during a first time span after administering a contrast agent,
- feeding the plurality of MRI images to a prediction model (PM),
- generating one or more predicted MRI images (4) showing the examination region during a second time span by means of the prediction model (PM), the second time span following the first time span chronologically,
- displaying and/or outputting the one or more predicted MRI images (4) and/or storing the one or more predicted MRI images (4) in a data storage medium,
the prediction model (PM) having been trained by means of supervised learning to predict, on the basis of reference MRI images, at least some of which show an examination region during a first time span after administering a contrast agent, one or more reference MRI images showing the examination region during a second time span, the second time span following the first time span chronologically,
the examination region being a liver or a portion of a liver of a human,
the contrast agent being a hepatobiliary contrast agent, the first time span starting within a time span of from one minute to one second before the administration of the contrast agent or with the administration of the contrast agent, and lasting for a time span of from 2 minutes to 15 minutes from the administration of the contrast agent, the second time span starting at least 10 minutes after the administration of the contrast agent.

12. Use of a contrast agent in a computer-implemented MRI method, after a contrast agent has been administered and has spread in an examination region, the computer-implemented MRI method comprising the following steps:
- generating a plurality of MRI images (1, 2, 3) of the examination region during a first time span,
- feeding the generated MRI images (1, 2, 3) to a prediction model (PM),
- receiving one or more predicted MRI images (4) showing the examination region during a second time span, as output from the prediction model (PM), the second time span following the first time span chronologically,
- displaying and/or outputting the one or more predicted MRI images (4) and/or storing the one or more predicted MRI images (4) in a data storage medium,
the prediction model (PM) having been trained by means of supervised learning to predict, on the basis of reference MRI images, at least some of which show an examination region during a first time span after administering a contrast agent, one or more reference MRI images showing the examination region during a second time span, the second time span following the first time span chronologically,
the examination region being a liver or a portion of a liver of a human,
the contrast agent being a hepatobiliary contrast agent, the first time span starting within a time span of from one minute to one second before the administration of the contrast agent or with the administration of the contrast agent, and lasting for a time span of from 2 minutes to 15 minutes from the administration of the contrast agent, the second time span starting at least 10 minutes after the administration of the contrast agent.

13. Kit comprising a contrast agent and a computer program product according to Claim 11, the contrast agent being a hepatobiliary MRI contrast agent.

## Revendications

1. Procédé mis en œuvre par ordinateur, comprenant les étapes suivantes :
- réception d'une pluralité d'enregistrements IRM, au moins une partie des enregistrements IRM montrant une zone d'examen pendant une première période après une application d'un agent de contraste,
- acheminement de la pluralité d'enregistrements IRM (1, 2, 3) à un modèle de prédiction (PM),
- génération d'un ou plusieurs enregistrements IRM prédits (4), qui montrent la zone d'examen pendant une deuxième période, par le modèle de prédiction (PM), la deuxième période suivant chronologiquement la première période,
- affichage et/ou délivrance en sortie dudit ou des plusieurs enregistrements IRM prédits (4) et/ou enregistrement dudit ou des plusieurs enregistrements IRM prédits (4) dans une mémoire de données
le modèle de prédiction (PM) ayant été entraîné au moyen d'un apprentissage surveillé, à l'aide d'enregistrements IRM de référence dont au moins une partie montre une zone d'examen pendant une première période après une application d'un agent de contraste, à prédire un ou plusieurs enregistrements IRM de référence, qui montrent la zone d'examen pendant une deuxième période, la deuxième période suivant chronologiquement la première période,
la zone d'examen étant un foie ou une partie d'un foie d'un être humain,
l'agent de contraste étant un agent de contraste hépatobiliaire,
la première période commençant dans une période d'une minute à une seconde avant l'application de l'agent de contraste ou avec l'application de l'agent de contraste, et une période de 2 minutes à 15 minutes s'écoulant à partir de l'application de l'agent de contraste,
la deuxième période commençant au moins 10 minutes après l'application de l'agent de contraste.

2. Procédé selon la revendication 1, au moins l'un des enregistrements IRM (1, 2, 3) reçus montrant la zone d'examen avant l'application de l'agent de contraste et au moins l'un des enregistrements IRM (1, 2, 3) reçus montrant la zone d'examen après l'application de l'agent de contraste.

3. Procédé selon la revendication 2, la première période étant choisie de telle sorte qu'elle montre la zone d'examen dans différentes phases, les phases comprenant une phase native, une phase artérielle, une phase veineuse portale et une phase tardive, au moins un enregistrement IRM étant reçu, lequel montre la zone d'examen dans la phase native, et au moins un enregistrement IRM étant reçu, lequel montre la zone d'examen pendant la phase artérielle, et au moins un enregistrement IRM étant reçu, lequel montre la zone d'examen dans la phase veineuse portale, et au moins un enregistrement IRM étant reçu, lequel montre la zone d'examen dans la phase tardive.

4. Procédé selon l'une des revendications 1 à 3, les enregistrements IRM (1, 2, 3) reçus montrant un foie ou une partie d'un foie d'un mammifère avant un instant TP0 et pendant une période de TP0 à TP1 et/ou pendant une période de TP1 à TP2 et/ou pendant une période de TP2 à TP3 et/ou pendant une période de TP3 à TP4, à l'instant TP0 l'agent de contraste étant appliqué par voie intraveineuse sous forme de bolus et parvenant ensuite dans les cellules du foie par le biais des artères hépatiques et des veines hépatiques, à l'instant TP1 l'agent de contraste atteignant une concentration maximale dans les artères hépatiques, à l'instant TP2 une intensité de signal, générée par l'agent de contraste dans les veines hépatiques, adoptant une valeur qui est aussi élevée qu'une intensité de signal générée par l'agent de contraste dans les artères hépatiques, à l'instant TP3 l'agent de contraste atteignant une concentration maximale dans les veines hépatiques, à l'instant TP4 une intensité de signal, générée par l'agent de contraste dans les cellules du foie, adoptant une valeur qui est aussi élevée qu'une intensité de signal générée par l'agent de contraste dans les veines hépatiques.

5. Procédé selon l'une des revendications 1 à 4, la première période à partir de l'application de l'agent de contraste durant une période de 2 minutes à 13 minutes, de préférence de 3 minutes à 10 minutes.

6. Procédé selon l'une des revendications 1 à 5, la deuxième période se trouvant au sein d'une phase hépatobiliaire.

7. Procédé selon l'une des revendications 1 à 6, la deuxième période commençant au moins 20 minutes après l'application de l'agent de contraste.

8. Procédé selon l'une des revendications 1 à 7, le modèle de prédiction (PM) étant un réseau neuronal artificiel.

9. Procédé selon l'une des revendications 1 à 8, l'agent de contraste étant du Gd-EOB-DTPA ou du Gd-BOPTA.

10. Système (10), comprenant
* une unité de réception (11),
* une unité de commande et de calcul (12) et
* une unité de sortie (13),
- l'unité de commande et de calcul (12) étant configurée pour demander à l'unité de réception (11) de recevoir une pluralité d'enregistrements IRM, au moins une partie des enregistrements IRM (1, 2, 3) montrant une zone d'examen pendant une première période après une application d'un agent de contraste,
- l'unité de commande et de calcul (12) étant configurée pour, à l'aide de la pluralité d'enregistrements IRM (1, 2, 3) reçus, prédire un ou plusieurs enregistrements IRM (4) au moyen d'un modèle de prévision, le ou les plusieurs enregistrements IRM prédits (4) montrant la zone d'examen pendant une deuxième période, la deuxième période suivant chronologiquement la première période,
- l'unité de commande et de calcul (12) étant configurée pour demander à l'unité de sortie (13) d'afficher, de délivrer en sortie ou d'enregistrer dans une mémoire de données ledit ou les plusieurs enregistrements IRM prédits (4),
le modèle de prédiction (PM) ayant été entraîné au moyen d'un apprentissage surveillé, à l'aide d'enregistrements IRM de référence dont au moins une partie montre une zone d'examen pendant une première période après une application d'un agent de contraste, à prédire un ou plusieurs enregistrements IRM de référence, qui montrent la zone d'examen pendant une deuxième période, la deuxième période suivant chronologiquement la première période,
la zone d'examen étant un foie ou une partie d'un foie d'un être humain,
l'agent de contraste étant un agent de contraste hépatobiliaire,
la première période commençant dans une période d'une minute à une seconde avant l'application de l'agent de contraste ou avec l'application de l'agent de contraste, et une période de 2 minutes à 15 minutes s'écoulant à partir de l'application de l'agent de contraste,
la deuxième période commençant au moins 10 minutes après l'application de l'agent de contraste.

11. Produit de programme informatique, comprenant un programme informatique qui peut être chargé dans une mémoire de travail d'un système informatique et qui peut y amener le système informatique à exécuter les étapes suivantes :
- réception d'une pluralité d'enregistrements IRM (1, 2, 3), au moins une partie des enregistrements IRM (1, 2, 3) montrant une zone d'examen pendant une première période après une application d'un agent de contraste,
- acheminement de la pluralité d'enregistrements IRM à un modèle de prédiction (PM),
- génération d'un ou plusieurs enregistrements IRM prédits (4), qui montrent la zone d'examen pendant une deuxième période, par le modèle de prédiction (PM), la deuxième période suivant chronologiquement la première période,
- affichage et/ou délivrance en sortie dudit ou des plusieurs enregistrements IRM prédits (4) et/ou enregistrement dudit ou des plusieurs enregistrements IRM prédits (4) dans une mémoire de données,
le modèle de prédiction (PM) ayant été entraîné au moyen d'un apprentissage surveillé, à l'aide d'enregistrements IRM de référence dont au moins une partie montre une zone d'examen pendant une première période après une application d'un agent de contraste, à prédire un ou plusieurs enregistrements IRM de référence, qui montrent la zone d'examen pendant une deuxième période, la deuxième période suivant chronologiquement la première période,
la zone d'examen étant un foie ou une partie d'un foie d'un être humain,
l'agent de contraste étant un agent de contraste hépatobiliaire,
la première période commençant dans une période d'une minute à une seconde avant l'application de l'agent de contraste ou avec l'application de l'agent de contraste, et une période de 2 minutes à 15 minutes s'écoulant à partir de l'application de l'agent de contraste,
la deuxième période commençant au moins 10 minutes après l'application de l'agent de contraste.

12. Utilisation d'un agent de contraste dans un procédé d'IRM mis en œuvre par ordinateur, après qu'un agent de contraste ait été appliqué et se soit réparti dans une zone d'examen, le procédé d'IRM mis en œuvre par ordinateur comprenant les étapes suivantes :
- génération d'une pluralité d'enregistrements IRM (1, 2, 3) de la zone d'examen pendant une première période,
- acheminement des enregistrements IRM (1, 2, 3) générés à un modèle de prédiction (PM),
- réception d'un ou plusieurs enregistrements IRM prédits (4), qui montrent la zone d'examen pendant une deuxième période, en tant que sortie du modèle de prédiction (PM), la deuxième période suivant chronologiquement la première période,
- affichage et/ou délivrance en sortie dudit ou des plusieurs enregistrements IRM prédits (4) et/ou enregistrement dudit ou des plusieurs enregistrements IRM prédits (4) dans une mémoire de données,
le modèle de prédiction (PM) ayant été entraîné au moyen d'un apprentissage surveillé, à l'aide d'enregistrements IRM de référence dont au moins une partie montre une zone d'examen pendant une première période après une application d'un agent de contraste, à prédire un ou plusieurs enregistrements IRM de référence, qui montrent la zone d'examen pendant une deuxième période, la deuxième période suivant chronologiquement la première période,
la zone d'examen étant un foie ou une partie d'un foie d'un être humain,
l'agent de contraste étant un agent de contraste hépatobiliaire,
la première période commençant dans une période d'une minute à une seconde avant l'application de l'agent de contraste ou avec l'application de l'agent de contraste, et une période de 2 minutes à 15 minutes s'écoulant à partir de l'application de l'agent de contraste,
la deuxième période commençant au moins 10 minutes après l'application de l'agent de contraste.

13. Kit comprenant un agent de contraste et un produit de programme informatique selon la revendication 11, l'agent de contraste étant un agent de contraste d'IRM hépatobiliaire.
